# EUROPEAN PATENT APPLICATION

(11) **EP 1 420 026 A2**
(43) Date of publication of application: **19.05.2004**
(21) Application number: 03025643.2
(22) Date of filing: 20.06.2000
(51) Int. Cl.: C07J 17/00, C07J 51/00, A23L 1/30

(54) **Use of conjugates of phytosterol or phytostanol with ascorbic acid in treating or preventing cardiovascular disease, and compositions thereof**

(30) Priority: 23.06.1999 US 339903
(62) Divisional of application: 00938426.4
(71) Applicant: Forbes Medi-Tech Inc., Vancouver, British Columbia V6C 2T8 (CA)
(72) Inventor: Kutney, James, P., Vancouver, British Columbia, V6L 3C7 (CA); Chen, Honming, Vancouver, British Columbia, V6T 1R9 (CA); Milanova, Radka, K., Vancouver, British Columbia, V6G 1E1 (CA); Ding, Yangbing, Vancouver, British Columbia, V6T 1R9 (CA); Hou, Duanjie, Vancouver, British Columbia, V6T 1R9 (CA)
(74) Representative: Jones, Helen Marjorie Meredith

(57) **Abstract**

Novel phytosterol and/or phytostanol derivatives, including salts thereof, are represented by general formulae: (I), (II), (III), wherein R is a phytosterol or phytostanol moiety, R2 is derived from ascorbic acid and R3 is hydrogen or any metal, alkali earth metal, or alkali metal. These derivatives are effective in treating and preventing cardiovascular disease and its underlying conditions including atherosclerosis and hyperlipedemia.

## Description

### FIELD OF THE INVENTION

This present invention relates to the field of phytosterols and phytostanols and their use in treating and preventing cardiovascular disease and other disorders.

### BACKGROUND OF THE INVENTION

While recent advances in science and technology are helping to improve quality and add years to human life, the prevention of atherosclerosis, the underlying cause of cardiovascular disease ("CVD") has not been sufficiently addressed. Atherosclerosis is a degenerative process resulting from an interplay of inherited (genetic) factors and environmental factors such as diet and lifestyle. Research to date suggest that cholesterol may play a role in atherosclerosis by forming atherosclerotic plaques in blood vessels, ultimately cutting off blood supply to the heart muscle or alternatively to the brain or limbs, depending on the location of the plaque in the arterial tree (1,2). Overviews have indicated that a 1% reduction in a person's total serum cholesterol yields a 2% reduction in risk of a coronary artery event (3). Statistically, a 10% decrease in average serum cholesterol (e.g. from 6.0 mmol/L to 5.3 mmol/L) may result in the prevention of 100,000 deaths in the United States annually (4).

Sterols are naturally occurring compounds that perform many critical cellular functions. Phytosterols such as campesterol, stigmasterol and beta-sitosterol in plants, ergosterol in fungi and cholesterol in animals are each primary components of cellular and sub-cellular membranes in their respective cell types. The dietary source of phytosterols in humans comes from plant materials i.e. vegetables and plant oils. The estimated daily phytosterol content in the conventional westem-type diet is approximately 60-80 milligrams in contrast to a vegetarian diet which would provide about 500 milligrams per day.

Phytosterols have received a great deal of attention due to their ability to decrease serum cholesterol levels when fed to a number of mammalian species, including humans. While the precise mechanism of action remains largely unknown, the relationship between cholesterol and phytosterols is apparently due in part to the similarities between the respective chemical structures (the differences occurring in the side chains of the molecules). It is assumed that phytosterols displace cholesterol from the micellar phase and thereby reduce its absorption or possibly compete with receptor and/or carrier sites in the cholesterol absorption process.

Over forty years ago, Eli Lilly marketed a sterol preparation from tall oil and later from soybean oil called Cytellin™ which was found to lower serum cholesterol by about 9% according to one report (5). Various subsequent researchers have explored the effects of sitosterol preparations on plasma lipid and lipoprotein concentrations (6) and the effects of sitosterol and campesterol from soybean and tall oil sources on serum cholesterols (7). A composition of phytosterols which has been found to be highly effective in lowering serum cholesterol is disclosed in US Patent Serial No. 5,770,749 to Kutney et al. and comprises no more than 70% by weight beta-sitosterol, at least 10% by weight campesterol and stigmastanol (beta-sitostanol). It is noted in this patent that there is some form of synergy between the constituent phytosterols, affording even better cholesterol-lowering results than had been previously achieved.

Despite the obvious and now well recorded advantages of phytosterols, not only in the treatment of CVD and its underlying conditions such as hypercholesterolemia, hyperlipidemia, atherosclerosis, hypertension, thrombosis but in the treatment of other diseases such as Type II diabetes, dementia cancer and aging, the administration of phytosterols and the incorporation thereof into foods, pharmaceuticals and other delivery vehicles has been complicated by the fact that they are highly hydrophobic (i.e.they have poor water solubility). Studies have investigated how the form (for example crystalline, suspension, granular) in which the phytosterols are dosed impacts on their ability to lower serum cholesterol levels. As phytosterols are highly hydrophobic, they do not dissolve to any appreciable extent in the micellar phase in the digestive tract and therefore are not capable of efficiently blocking cholesterol absorption. Oils and fats are capable to a limited but not satisfactory degree of dissolving free phytosterols. Since only solubilized phytosterols inhibit absorption of cholesterol, adaptations must be made.

Early research focused on grinding or milling the phytosterols in order to enhance their solubility (US Patent Serial Nos: 3,881,005 and 4,195,084 both to Eli Lilly). In addition, researchers have looked to the esterification of phytosterols in order to enhance their solubility. German Patent 2035069/January 28, 1971 (analogous to US Patent No. 3,751,569) describes the addition of phytosterol fatty acid esters to cooking oil. The esterification is carried out between a free sterol and a fatty acid anhydride, with perchloric acid as the catalyst. The significant drawback to this process, along with others, is the use of non-food grade catalysts and reagents.

US Patent Serial no. 4,588,717 to the David E. Mitchell Medical Research Institiute describes a vitamin supplement which comprises a fatty acid ester of a phytosterol, wherein the fatty acid ester has from about 18 to 20 carbon atoms in the main carbon chain.

US Patent Serial No. 5,502,045 to Raision Tehtaat Oy AB describes the preparation of a beta-sitostanol fatty acid ester mixture. Although the attempt of this patent is to produce a soluble and stable phytostanol delivery system, there are some problems with the long term stability of these "fatty acid" esterified products due to the ultimate oxidation of the unsaturated fatty acid moiety.

Accordingly, the provision of a stable water-soluble phytosterol/phytostanol derivative which could be administered orally and which could be incorporated without further modification into delivery vehicles would be highly desirable and has not heretofore been satisfactorily achieved.

It is an object of the present invention to obviate or mitigate the above disadvantages.

### SUMMARY OF THE INVENTION

The present invention provides novel derivatives comprising phytosterol and/or phytostanol and ascorbic acid, including salts thereof, and represented by the general formulae: 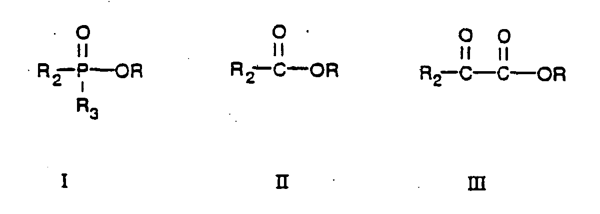 wherein R is a phytosterol or phytostanol moiety; R2 is derived from ascorbic acid and R3 is hydrogen or any metal, alkali earth metal, or alkali metal.

The present invention also comprises processes of preparing the novel derivatives having the above noted formulae.

The present invention further comprises a composition for treating or preventing CVD and its underlying conditions including atherosclerosis, hypercholesterolemia, hyperlipidemia, hypertension, thrombosis, and related diseases such as Type II diabetes, as well as other diseases that include oxidative damage as part of the underlying disease process such as dementia, Alzheimer's disease, aging, and cancer which comprises one or more derivatives of phytosterols and/or phytostanols and ascorbic acid, having one or more of the above noted formulae, and a pharmaceutically acceptable carrier therefor.

The present invention further provides foods, beverages and nutraceuticals supplemented with derivatives of phytosterols and/or phytostanols and ascorbic acid, having one or more of the above noted formulae.

The present invention further provides a method for treating or preventing CVD and its underlying conditions including atherosclerosis, hypercholesterolemia, hyperlipidemia, hypertension, thrombosis, and related diseases such as Type II diabetes, as well as other diseases that include oxidative damage as part of the underlying disease process such as dementia, aging, and cancer by administering to an animal derivatives of phytosterols and/or phytostanols and ascorbic acid, having one or more of the above noted formulae.

The phytosterol/phytostanol/ascorbic acid derivatives and salts thereof of the present invention have numerous advantages over non-modifed phytosterol/stanol compositions previously known and described in the art. In particular, it has been found that solubility in aqueous solutions such as water is improved thereby allowing oral administration per se without any further enhancements or modifications. Accordingly, the derivatives of the present invention can be prepared and used as such or they can be easily incorporated into foods, beverages, pharmaceuticals and nutraceuticals regardless of whether these "vehicles" are water-based. This enhanced solubility generally translates into lower administration dosages of the derivatives in order to achieve the desired therapeutic effect.

A second advantage of the derivatives of the present invention is that there may be an additive or synergistic therapeutic effect, both in lowering serum cholesterol and as an anit-oxidant, between the phytosterol/stanol component and the ascorbic acid. These effects and other significant advantages are described in more detail below.

### BRIEF DESCRIPTION OF THE DRAWINGS

The present invention is illustrated by way the following non-limiting drawings in which:
Figure 1 is a schematic showing a process of preparing phytostanol-phosphate-ascorbate and its sodium salt;
Figure 2 is a schematic showing a process of preparing phytostanol-carbonate-ascorbate and its sodium salt;
Figure 3 is a schematic showing a process of preparing phytostanol-oxalate-ascorbate and its sodium salt;
Figure 4 is a graph showing the effects of Vitosterol on plasma cholesterol levels in cholesterol-fed apo E-KO mice;
Figure 5 is a graph showing the effects of Vitosterol on plasma triglyceride levels in cholesterol-fed apo E-KO mice;

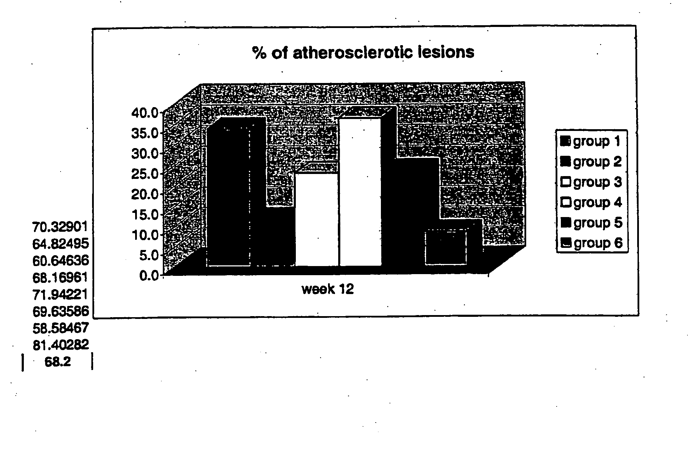

Figure 6 is a graph showing the effects of Vitosterol on mouse body weight; and
Figure 7 is a graph showing the effects of Vitosterol on the percentage of atherosclerotic formulae: 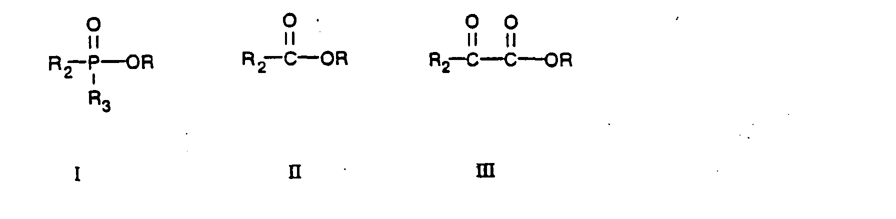
wherein R is a phytosterol or phytostanol moiety, R2 is derived from ascorbic acid and R3 is hydrogen or any metal, alkali earth metal, or alkali metal. The components of the derivative will be described in more detail below. It should be noted that, throughout this disclosure, the terms "derivative", "structure" and "analogue" are used interchangeably to describe the novel unitary compound which links both a phytosterol or phytostanol and ascorbic acid.

### Phytosterols/Phytostanols

As used herein, the term "phytosterol' includes all phytosterols without limitation, for example: sitosterol, campesterol, stigmasterol, brassicasterol, desmosterol, chalinosterol, poriferasterol, clionasterol and all natural or synthesized forms and derivatives thereof, including isomers. The term "phytostanol" includes all saturated or hydrogenated phytosterols and all natural or synthesized forms and derivatives thereof, including isomers. It is to be understood that modifications to the phytosterols and phytostanols i.e. to include side chains also falls within the purview of this invention. It is also to be understood that, when in doubt throughout the specification, the term "phytosterol" encompasses both phytosterol and phytostanol i.e. the terms may be used interchangeably unless otherwise specified.

The phytosterols and phytostanols for use in forming derivatives in accordance with this invention may be procured from a variety of natural sources. For example, they may be obtained from the processing of plant oils (including aquatic plants) such as com oil and other vegetable oils, wheat germ oil, soy extract, rice extract, rice bran, rapeseed oil, sunflower oil, sesame oil and fish (and other marine-source) oils. The present invention poriferasterol, clionasterol and all natural or synthesized forms and derivatives thereof, including isomers. The term "phytostanol" includes all saturated or hydrogenated phytosterols and all natural or synthesized forms and derivatives thereof, including isomers. It is to be understood that modifications to the phytosterols and phytostanols i.e. to include side chains also falls within the purview of this invention. it is also to be understood that, when in doubt throughout the specification, the term "phytosterol" encompasses both phytosterol and phytostanol i.e. the terms may be used interchangeably unless otherwise specified.

The phytosterols and phytostanols for use in forming derivatives in accordance with this invention may be procured from a variety of natural sources. For example, they may be obtained from the processing of plant oils (including aquatic plants) such as com oil and other vegetable oils, wheat germ oil, soy extract, rice extract, rice bran, rapeseed oil, sunflower oil, sesame oil and fish (and other marine-source) oils. The present invention is not to be limited to any one source of phytsterols. US Patent Serial No. 4,420,427 teaches the preparation of sterols from vegetable oil sludge using solvents such as methanol. Alternatively, phytosterols and phytostanols may be obtained from tall oil pitch or soap, by-products of forestry practises as described in US Patent Serial No.5,770,749, incorporated herein by reference.

In one preferred form, the derivative of the present invention is formed with naturally-derived or synthesized beta-sitosterol, campestanol, sitostanol and campesterol and each of these derivatives so formed may then be admixed a composition prior to delivery in various ratios. In another preferred form, the derivative of the present invention is formed with naturally-derived or synthesized sitostanol or with naturally derived or synthesized campestanol or mixtures thereof.

### R2

R2 comprises ascorbic acid or any derivative thereof. What is achieved within the scope of the present invention is the creation of a new structure or compound wherein a phytosterol or phytostanol moiety is chemically linked to ascorbic acid. The union benefits and enhances the both parts of this new structure. The phytosterol moiety, formerly poorly soluble, becomes, as part of the new derivative, much more readily soluble in aqueous and non-aqueous media such as oils and fats. Accordingly, administration of the phytosterol becomes possible without any further enhancements to modify its delivery.

For many years, it has been recognized that L-ascorbic acid (commonly known as vitamin C) is a vital part of balanced human nutrition and plays a role as a physiological antioxidant. However, ascorbic acid is the least stable vitamin with which to work since it reacts extremely easily with atmospheric oxygen yielding dehydroascorbic acid which further and readily decomposes into compounds void of vitamin C efficacy. It is believed that the new structure of the present invention "protects" ascorbic acid from such decomposition. Furthermore, it is believed that the anti-oxidative and other therapeutic effects of ascorbic acid are enhanced in a synergistic or additive fashion as a unitary compound formed with phytosterol or phytostanol. These advantages have not heretofore been appreciated or explored.

### Derivative Formation

### a) Ester Formation

There are many processes by which novel structures comprising phytosterols and/or phytostanols and ascorbic acid can be formed. In general, the selected phytosterol or stanol (or halophosphate, halocarbonate or halo-oxalate derivatives thereof) and ascorbic acid are mixed together under reaction conditions to permit condensation of the "acid" moiety with the "alcohol" (phytosterol). These conditions are the same as those used in other common esterification reactions such as the Fisher esterification process in which the acid component and the alcohol component are allowed to react directly or in the presence of a suitable acid catalyst such as mineral acid, sulfuric acid, phosphoric acid, p-toluenesulfonic acid. The organic solvents generally employed in such esterification reactions are ethers such as diethyl ether, tetrahydrofuran, or benzene, toluene or similar aromatic solvents and the temperatures can vary from room to elevated temperatures depending on the reactivity of the reactants undergoing the reaction.

In a preferred embodiment, the process to form the ester derivative comprises "protecting" the hydroxyl groups of the ascorbic acid or derivatives thereof as esters (for example, as acetate esters) or ethers (for example, methyl ethers) and then condensing the protected ascorbic acid with the phytosterol/phytostanol halophospahte, halocarbonate or halo-oxalate under suitable reaction conditions. In general, such condensation reactions are conducted in an organic solvent such as diethyl ether, tetrahydrofuran, or benzene, toluene or similar aromatic solvents. Depending on the nature and reactivity of the reactants, the reaction temperatures may vary from low (-15°C) to elevated temperatures.

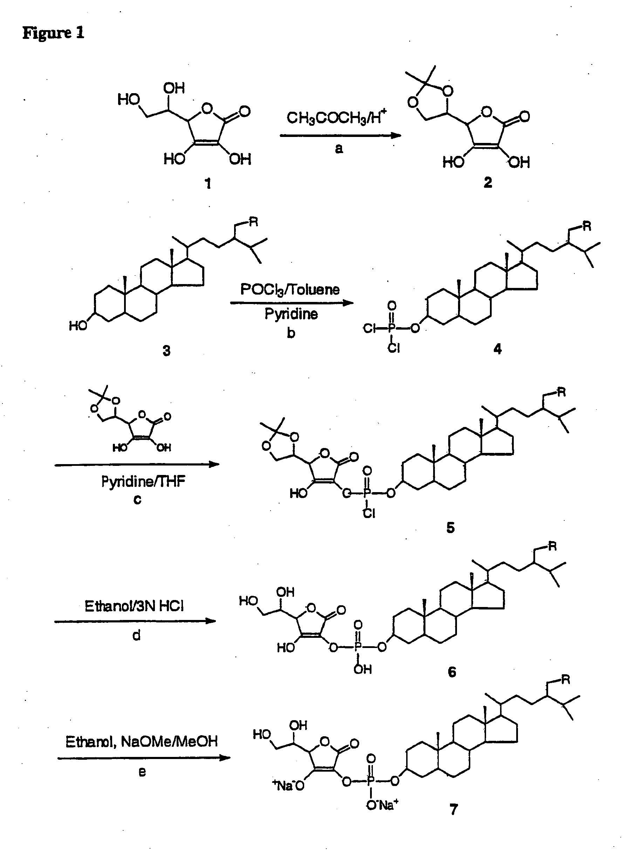

Figure 1 is a schematic showing the formation of the "protected" ascorbic acid (step a), the formation of the intermediary chlorophosphate/stanol derivative (step b), and the condensation reaction (alternatively steps c or d) yielding one of novel derivatives of the present invention based on formula I: phytostanol-phosphate-ascorbate (noted as structure 6).

In more detail, the process shown in Figure 1 is as follows: ascorbic acid is initially protected from decomposition by the formation of 5,6-isopropylidene-ascorbic acid (structure 2). This can be achieved by mixing acetone with ascorbic acid and an acidic catalyst such as sulfuric acid or hydrochloric acid under suitable reaction conditions (refer to Example 1 below). Phytostanol chlorophosphate (structure 4) is prepared by forming a solution of phytostanol in toluene and pyridine (although other nitrogen bases such as aliphatic and aromatic amines may alternatively be used) and treating this solution with a phosphorus derivative such as phosphorus oxychloride. The residue so formed after filtration and concentration of the mother liquor is phytostanol chlorophosphate (structure 4). The latter is then mixed with 5,6-isopropylidene-ascorbic acid and, after the addition of a suitable alcohol such as ethanol and HCl (step d), concentrated. Alternatively. pyridine/THF may be added (step c) and the product concentrated. After final washing and drying (step e), the resultant novel product of both steps c or d is phytostanol-phosphate-ascorbate (structure 6).

In another preferred form of the process of the present invention, ascorbic acid is protected at the hydroxyl sites not as 5,6-isopropylidene-ascorbic acid but as esters (for example as acetates, phosphates and the like..). The latter may then be condensed with phytosterols or phytostanols, derivatized as described above, using known esterification methods ultimately to produce the structures of the present invention. The formation of mono and diphosphates of ascorbic acid is described thoroughly in the literature. For example, US Patent Serial No. 4,939,128 to Kato et al., the contents of which are incorporated herein by reference, teaches the formation of phosphoric acid esters of ascorbic acid. Similarly, US Patent Serial No. 4,999,437 to Dobler et al., the contents of which are also fully incorporated herein by reference, describes the preparation of ascorbic acid 2-phosphate. In Dobler et al., the core reaction of phosphorylating ascorbic acid or ascorbic acid derivatives with POCl3 in the presence of tertiary amines (described in German Laid Open Application DOS 2,719,303) is improved by adding to the reaction solution a magnesium compound, preferably an aqueous solution of a magnesium compound. Any of these known ascorbic acid derivatives can be used within the scope of the present invention.

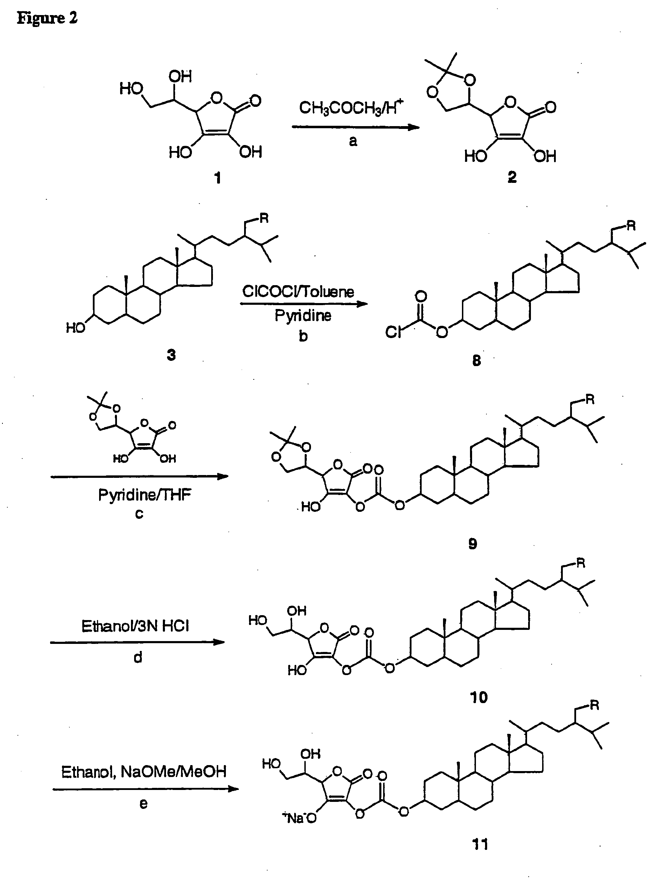

Figure 2 is a schematic showing the formation of the "protected" ascorbic acid (step a), the formation of the intermediary chlorocarbonate/stanol derivative (step b), and the condensation reaction (optionally steps c or d) yielding structure 9 (10 is the same), one of novel derivatives of the present invention based on formula II: phytostanol-carbonate-ascorbate. These chlorocarbonate derivatives may be prepared by the same process outlined in detail above with respect to Figure 1; however, the phosphorus oxylchloride is replaced (as shown in step b of Figure 2) by phosgene.

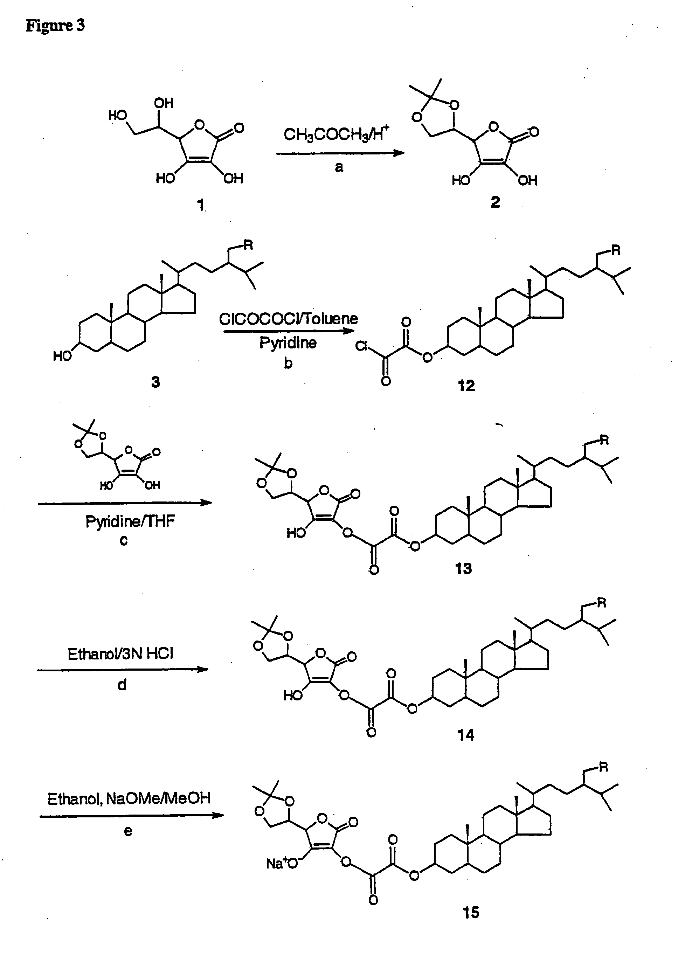

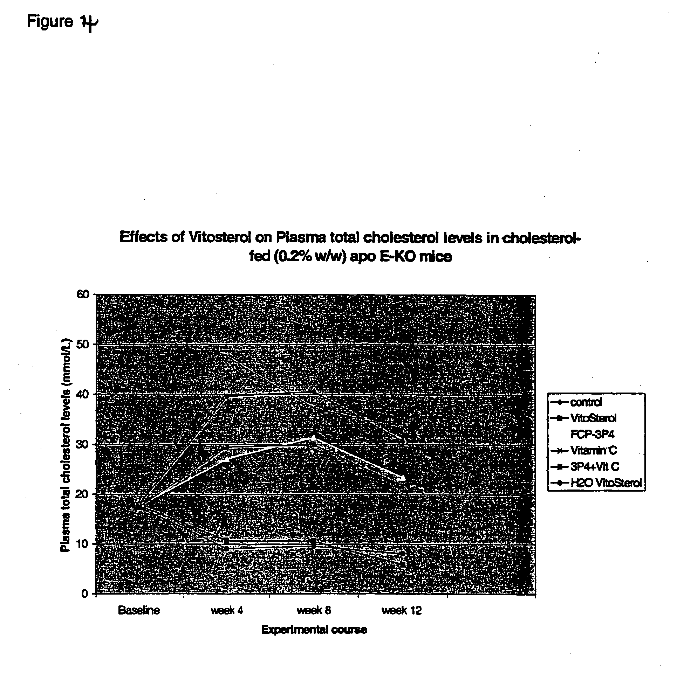

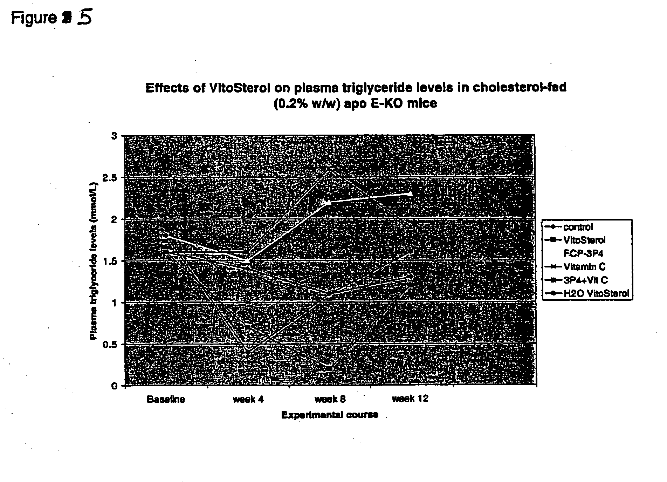

Figure 3 is a schematic showing the formation of the "protected" ascorbic acid (step a), the formation of the intermediary chloro-oxalate/stanol derivative (step b), and the condensation reaction (optionally steps c or d) yielding a novel structure 13 (same as 14), one of novel derivatives of the present invention based on formula III: phytostanol-oxalate-ascorbate (noted as structure 14). These chloro-oxalate derivatives may be prepared by the same process outlined in detail above with respect to Figure 1; however, the phosphorus oxylchloride is replaced (as shown in step b of Figure 3) by oxalyl chloride.

### b) Salt Formation

The present invention encompasses not only the parent structures comprising phytosterols or phytostanols and ascorbic acid (for example, those preferred structures shown as structures 5 and 6 in Figure 1, structures 9 an 10 in Figure 2 and structures 13 and 14 in Figure 3) but also the salts thereof. These salts are even more water soluble than the corresponding parent compounds and therefore their efficacy and evaluation both *in vitro* and *in vivo* is much improved.

Salt formation of the derivatives of the present invention can be readily performed by treatment of the parent compound with a series of bases (for example, sodium methoxide or other metal alkoxides) to produce the corresponding alkali metal salts. Other metal salts of calcium, magnesium, manganese, copper, zinc, and the like can be generated by reacting the parent with suitable metal alkoxides. With respect to formula I, R3 represents either hydrogen (parent compound) or any metal, alkali earth metal, or alkali metal (the salt).

### c) Reduction by Catalytic (Hydrogenation) and Chemical Methods

Optionally, the phytosterol derivatives of the present invention or the constituent moieties thereof (either the phytosterol or the ascorbic acid) prior to or after derivative formation may be hydrogenated or saturated. The hydrogenation of heterocyclic ring systems to the partially or fully reduced analogues is a well known process. For example, the catalytic and/or chemical reduction of the ring of ascorbic acid to the corresponding dihydro analogue is readily accomplished under an atmosphere of hydrogen and a metal catalyst such as platinum, palladium or Raney Nickel. In general, this reduction is performed in an organic solvent such as ethanol, ethyl acetate or similar media and either under atmospheric pressure or at a low pressure (3-5 psi) at room temperature or slightly elevated temperatures.

The chemical reductions of such systems involve reduction with a family of "hydride" reagents such as sodium borohydride, lithium aluminum hydride and their analogues. These reductions are generally performed in an anhydrous inert medium involving ethyl ether, tetrahydrofuran, dioxane, or benzene, toluene or similar aromatic solvents at room to reflux temperatures.

Similar catalytic or chemical processes can be applied to all of the phytosterol analogues of the present invention. Accordingly, the present invention includes within its scope all fully or partially reduced derivatives wherein the ring of ascorbic acid is partially or fully reduced and/or wherein the phytosterol moiety is fully or partially hydrogenated.

### Derivatives

The present invention comprises all derivatives comprising phytosterol and/or phytostanol and ascorbic acid, including salts thereof represented by the general formulae: 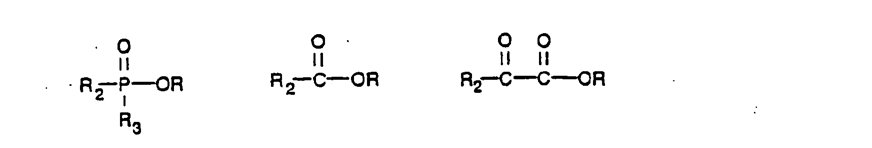 wherein R is a phytosterol or phytostanol moiety; R2 is derived from ascorbic acid and R3
is hydrogen or any metal, alkali earth metal, or alkali metal. Most preferably, the present invention comprises all halophosphate, halocarbonate and halo-oxalate/phytostanol/ascorbate derivatives as shown in Figures 1 through 3 as structures 5, 6, 7, 9, 10, 11 13 14 and 15. It is to be clearly understood; however, that these structures are only a selection of the many novel derivatives which fall within the
purview of formulae I, II and III. It is also to be understood that although sodium salts are shown in structures 7, 11 and 15, other salts are included within the scope of the invention, as described above.

### Combination of Derivatives with HMG-Coenzyme A Reductase inhibitors

In a further embodiment, the derivatives of the present invention may be combined, prior to administration, with one or more agents or compounds which inhibit cholesterol synthesis. These compounds include, but are not limited to 3-hydroxy-3-methyl glutaryl coenzyme-A (HMG-CoA) reductase inhibitors. The combination of these cholesterol synthesis-limiting compounds and the phytosterol derivatives of the present invention is synergistic and initiates and perpetuates both "systemic or enteric" and "extrinsic" effects.

Although the mechanism of action of the derivatives of the present invention is not certain, it is believed that cholesterol levels are lowered through a process called "biliary diversion", the uncoupling of communication between the biliary enterocytes and the hepatocytes. Enteric cholesterol production may increase but cholesterol is released in the bile and not reabsorbed through the enterocytes. Any cholesterol synthesis limiting compound would work in concert with these derivatives as the mechanism of the mechanism of the former is the decrease of enteric cholesterol synthesis and bile cholesterol secretion. As cholesterol synthesis is decreased, the phytosterol/cholesterol intestinal ratio increases which decreases cholesterol absorption and increases phytosterol transport via the enterocyte shuttle mechanism. The finding of this synergistic co-effect between the phytosterol derivatives of the present invention and the compounds which limit cholesterol synthesis, such as statins, is critically important as the dosage of these latter compounds may be significantly reduced when administered in conjunction with the derivatives described herein. It has recently been discovered that there are some critical side-effects to statins such as Lovastatin, so the dosage reduction afforded by the synergy with the derivatives described herein is particularly compelling. Examples of other statins with which the derivative of the present invention may be combined include: atorvastatin (Lipitor™), superstatin, simvastatin (Zocor™) and pravastatin (Pravachol™).

### Potential Advantages of Novel Phytosterol Analogues

The novel derivatives of the present invention, wherein ascorbic acid is attached to the phytosterol moiety affords many dietary and therapeutic advantages when compared to the use of phytosterols/phytostanols without such attachment. First and foremost, solubility of the novel derivatives is greatly enhanced, both in aqueous solutions and non-aqueous media such as oils and fats. With this greater solubility, effective dietary and therapeutic dosages and concomitantly costs, can be reduced. Secondly, it is very likely that there is even a synergistic or additive effect between the phytosterol moiety and the ascorbic acid, when united in one structure, in treating or preventing not only cardiovascular disease and its underlying conditions including atherosclerosis and hyperlipidemia but also diseases that have oxidative damage in their pathology such as cancer, aging, demetia and Alzheimer's. Thirdly, the formation of these derivatives allows the full potential of ascorbic acid to be realized while eliminating decomposition. Fourthly, these derivatives are heat stable (stable to oxidation and hydrolysis) which is essential for further processing in, for example, extruders and food processors.

### Delivery Systems

Although it is fully contemplated within the scope of the present invention that the derivatives may be administered to animals, particularly humans, directly and without any further modification, it is possible to take further steps to enhance delivery and ensure even distribution throughout the food, beverage, pharmaceutical, nutraceutical and the like to which they are added. It is to be understood; however, that these steps are purely optional. Such enhancement may be achieved by a number of suitable means such as, for example, solubilizing or dispersing the derivatives to form emulsions, solutions and dispersions or self-emulsifying systems; lyophilizing, spray drying, controlled precipitating, or a combination thereof; forming solid dispersions, suspensions, hydrated lipid systems; forming inclusion complexations with cyclodextrins; and using hydrotopes and formulations with bile acids and their derivatives.

Each of the techniques which may be used in accordance with the present invention are described below.

### Emulsions

Emulsions are finely divided or colloidal dispersions comprising two immiscible phases, e.g. oil and water, one of which (the internal or discontinuous phase) is dispersed as droplets within the other (external or discontinuous phase). Thus an oil-in-water emulsion consists of oil as the internal phase, and water as the discontinuous or external phase, the water-in-oil emulsion being the opposite. A wide variety of emulsified systems may be formed which comprise phytosterols or phytostanols derivatives including standard emulsions, microemulsions and those systems which are self-emulsifying (emulsify on exposure to agitated aqueous fluids such as gastric or intestinal fluids).

Generally, emulsions may include oil and water phases, emulsifiers, emulsion stabilizers and optionally preservatives, flavouring agents, pH adjusters and buffers, chelating agents, antifoam agents, tonicity adjusters and anti-oxidants. Suitable emulsifiers (wherein bracketed numerals refer to the preferred HLB values) include: anionic surfactants such as alcohol ether sulfates, alkyl sulfates (30-40), soaps (12-20) and sulfosuccinates; cationic surfactants such as quatemary ammonium compounds; zwitterionic surfactants such as alkyl betaine derivatives; amphoteric surfactants such as fatty amine sulfates, difatty alkyl triethanolamine derivatives (16-17); and nonionic surfactants such as the polyglycol ether derivatives of aliphatic or cycloaliphatic alcohols, saturated fatty acids and alkyphenols, water-soluble polyethyleneoxy adducts onto polypropylene glycol and alkyl polypropylene glycol, nonylphenol polyethoxyethanols, castor oil polyglycol ethers, polypropylene/polyethylene oxide adducts, tributylphenoxy-polyethoxyethanol, polyethylene glycol, octylphenoxy-polyethoxyethanol, lanolin alcohols, polyoxyethylated (POE) alkyl phenols(12-13), POE fatty amides, POE fatty alcohol ethers, POE fatty amines, POE fatty esters, poloxamers (7-19), POE glycol monoethers (13-16), polysorbates (17-19) and sorbitan esters (2-9). This list is not intended to be exhaustive as other emulsifiers are equally suitable.

Appropriate emulsion stabilizers include, but are not limited to, lyophilic colloids such as polysaccharides, acacia, agar, alginic acid, carrageenan, guar gum, karaya gum, tragacanth, xanthan gum; amphoterics (e.g. gelatin) and synthetic or semi-synthetic polymers (e.g. carbomer resins, cellulose ethers and esters, carboxymethyl chitin, polyethylene glycol-n (ethylene oxide polymer H(OCH2CH2)nOH); finely divided solids including clays (e.g. attapulgite, bentonite, hectorite, kaolin, magnesium aluminum silicate and montmorillonite), microcrystalline cellulose oxides and hydroxides (e.g. aluminum hydroxide. magnesium hydroxide and silica); and cybotactic promoters/gellants (including amino acids, peptides, proteins lecithin and other phospholipids and poloxamers).

Suitable anti-oxidants for use in the formation of emulsions include: chelating agents such as citric acid, EDTA, phenylalanine, phosphoric acid, tartaric acid and tryptophane; preferentially oxidized compounds such as ascorbic acid, sodium bisulfite and sodium sulfite; water soluble chain terminators such as thiols and lipid soluble chain terminators such as alkyl gallates, ascorbyl palmitate, t-butyl hydroquinone, butylated hydroxyanisole, butylated hydroxytoluene, hydroquinone, nordihydroguaiaretic acid and alpha-tocopherol. Suitable preservatives, pH adjustment agents, and buffers, chelating agents, osmotic agents, colours and flavouring agents are discussed hereinbelow under "Supensions", but are equally applicable with respect to the formation of emulsions.

The general preparation of emulsions is as follows: the two phases (oil and water) are separately heated to an appropriate temperature, the same in both cases, generally 5-10°C above the melting point of the highest melting ingredients in the case of a solid or semi-solid oil, or where the oil phase is liquid, a suitable temperature as determined by routine experimentation). Water-soluble components are dissolved in the aqueous (water) phase and oil-soluble components, are dissolved in the oil phase. To create an oil-in water emulsion, the oil phase is vigorously mixed into the aqueous phase to create a suitable dispersion and the product is allowed to cool at a controlled rate with stirring. A water-in-oil emulsion is formed in the opposite fashion i.e. the water phase is added to the oil phase. When hydrophilic colloids are a part of the system as emulsion stabilizers, a phase inversion technique may be employed whereby the colloid is mixed into the oil phase rather than the aqueous phase, prior to addition to the aqueous phase. In using any phytosterol or phytostanol derivatives, it is preferred to add these to the oil phase prior to heating.

Microemulsions, characterized by a particle size at least an order of magnitude smaller (10-100 nm) than standard emulsions and defined as "a system of water, oil and amphiphile which is a single optically isotropic and thermodynamically stable liquid" (8), may also be formed comprising phytosterol or phytostanol derivatives. In a preferred form, the microemulsion comprises a surfactant or surfactant mixture, a co-surfactant (usually a short chain alcohol) the chosen phytosterol or phytostanol derivatives, water and optionally other additives.

This system has several advantages as a delivery system for the derivatives of the present invention. Firstly, microemulsions tend to be created spontaneously, that is, without the degree of vigorous mixing required to form standard emulsions. from a commercial perspective, this simplifies the manufacturing process. Secondly, microemulsions may be sterilized using microfiltration techniques without breaking the microstructure due to the small diameter of the microdroplets. Thirdly, microemulsions are highly thermodynamically stable. Fourthly, microemulsions possess high solubilizing power which is particularly important as they can further enhance the solubilization of the derivatives.

Surfactant or surfactant mixtures which are suitable for use in the formation of microemulsions can be anionic, cationic, amphoteric or non-ionic and possess HLB (hydrophile-lipophile balance) values within the range of 1-20, more preferably in the ranges 2-6 and 8-17. Especially preferred agents are non-ionic surfactants, selected from the group consisting of polyglycol ether derivatives of aliphatic or cycloaliphatic alcohols, saturated fatty acids and alkyphenols, water-soluble polyethyleneoxy adducts onto polypropylene glycol and alkyl polypropylene glycol, nonylphenol polyethoxyethanols, castor oil polyglycol ethers, polypropylene/polyethylene oxide adducts, tributylphenoxy-polyethoxyethanol, polyethylene glycol, octylphenoxy-polyethoxyethanol, lanolin alcohols, polyoxyethylated (POE) alkyl phenols (12-13), POE fatty amides, POE fatty alcohol ethers, POE fatty amines, POE fatty esters, poloxamers (7-19), POE glycol monoethers (13-16), polysorbates (10-17) and sorbitan esters (2-9).

There are a number of methods known and used by those skilled in the art for making microemulsions. In a preferred method of forming microemulsions of the present invention, a surfactant, a co-surfactant, and phytosterol or phytostanol derivative (predissolved in a suitable proportion of an appropriate oil) are mixed and then titrated with water until a system of desired transparency is obtained.

In a further preferred embodiment, the formation of microemulsions may be achieved by mixing the phytosterol or phytostanol derivatives with hydrotropic agents and food-grade surfactants (refer to 9).

### Solutions and Dispersions

Phytosterol or phytostanol derivatives may be dissolved or dispersed in a suitable oil vehicle, with or without additional excipients, and used in this form, for example, in general food usage, in basting meats and fish, and for incorporation into animal feeds.

Suitable solubilizing agents include all food grade oils such as plant oils, marine oils (such as fish oil) and vegetable oils, monoglycerides, diglycerides, triglycerides, tocopherols and the like and mixtures thereof.

### Self-Emulsifying Systems

Phytosterol or phytostanol derivatives may be mixed with appropriate excipients, for example, surfactants, emulsion stabilizers (described above) and the like, heated (if necessary) and cooled to form a semi-solid product capable of forming a spontaneous emulsion on contact with aqueous media. This semi-solid product may be used in numerous other forms such as filler material in two-piece hard or soft gelatin capsules, or may be adapted for use in other delivery systems.

### Solid Dispersions

An alternative means of further increasing the solubility/dispersability of the derivuativeds in accordance with the present invention involves the use of solid dispersion systems. These dispersions may include molecular solutions (eutectics), physical dispersions or a combination of both.

For example, solid dispersions may typically be prepared by utilizing water-soluble polymers as carriers. Without limitation, these carriers may include, either alone or in combination: solid grade polyethylene glycols (PEG's), with or without the addition of liquid grade PEG'_{S}; polyvinylpyrrolidones or their co-polymers with vinyl acetate and cellulose ethers and esters. Other excipients, such as additional members of the glycol family e.g. propylene glycol, polyols, e.g. glycerol etc.. may also be included in the dispersions.

Solid dispersions may be prepared by a number of ways which are familiar to those in the art. These include, without limitation, the following methods:
(a) fusing the ingredients, followed by controlled cooling to allow solidification and subsequent mechanical grinding to produce a suitable powder. Alternatively, the molten (fused) dispersion may be sprayed into a stream of cooled air in a spray drier to form solid particles (prilling) or passed through an extruder and spheroniser to form solid masses of a controlled particle size. In a further alternative, the molten dispersion is filled directly into two-piece hard gelatin capsules;
(b) dissolving the ingredients in a suitable solvent system (organic, mixed organic, organic-aqueous) and then removing the solvents e.g. by evaporating at atmospheric pressure or in vacuo, spray drying, lyophilizing and the like; or, in a variation of the foregoing, and
(c) dissolving the ingredients in a suitable solvent system, subsequently precipitating them from solution by the use of an immiscible solvent in which the ingredients have little or no solubility, filtration, removing the solvent, drying and optionally grinding to provide a suitable powder form.

### Suspensions

Suspensions, which may be used to enhance further the solubility and/or dispersability of the derivatives , comprise a solid, perhaps finely divided, internal phase dispersed in an oily or aqueous external phase (the vehicle). In addition, the solid internal phase may be added to an emulsion as described above during its' formation to produce a delivery system having properties common to both suspensions and emulsions.

Numerous excipients, which are commonly used in the art, may be suitable for producing a suspension within the scope of the present invention. Typically, a suspension comprises an oily or aqueous vehicle, the dispersed (suspended) internal phase, dispersing and/or wetting agents (surfactants), pH adjustment agents/buffers, chelating agents, antioxidants, agents to adjust ionic strength (osmotic agents) colours, flavours, substances to stabilize the suspension and increase viscosity (suspending agents) and preservatives.

Appropriate vehicles include, but are not limited to: water, oils, alcohols, polyols, other edible or food grade compounds in which the phytosterol composition is partially or not soluble and mixtures thereof. Appropriate dispersing agents include, but are not limited to: lecithin; phospholipids; nonionic surfactants such as polysorbate 65, octoxynol-9, nonoxynol-10, polysorbate 60, polysorbate 80, polysorbate 40, poloxamer 235, polysorbate 20 and poloxamer 188; anionic surfactants such as sodium lauryl sulfate and docusate sodium; fatty acids, salts of fatty acids, other fatty acid esters, and mixtures thereof.

Agents/buffers for pH adjustment include citric acid and its salts, tartaric acid and its salts, phosphoric acid and its salts, acetic acid and its salts, hydrochloric acid, sodium hydroxide and sodium bicarbonate. Suitable chelating agents include edetates (disodium, calcium disodium and the like), citric acid and tartaric acid. Suitable antioxidants include ascorbic acid and its salts, ascorbyl palmitate, tocopherols (especially alpha-tocopherol), butylated hydroxytoluene, butylated hydroxyanisole, sodium bisulfite and metabisulfite. Suitable osmotic agents include monovalent, divalent and trivalent electrolytes, monosaccharides and disaccharides. Suitable preservatives include parabens (Me, Et, Pr, Bu and mixtures thereof), sorbic acid, thimerosal, quaternary ammonium salts, benzyl alcohol, benzoic acid, chlorhexidine gluconate and phenylethanol. Colours and flavours may be added as desired and may be selected from all natural, nature-identical and synthetic varieties.

### Hydrated Lipid Systems

In a further embodiment of the present invention, the solubility/dispersability of the derivtaives of the present invention may be further enhanced by the formation of phospholipid systems such as liposomes and other hydrated lipid phases, by physical inclusion. This inclusion refers to the entrapment of molecules without forming a covalent bond and is widely used to improve the solubility and subsequent dissolution of active ingredients.

Hydrated lipid systems, including liposomes, can be prepared using a variety of lipid and lipid mixtures, including phospholipids such as phosphatidylcholine (lecithin), phosphodiglyceride and sphingolipids, glycolipids, and the like. The lipids may preferably be used in combination with a charge bearing substances such as charge-bearing phospholipids, fatty acids, and potassium and sodium salts thereof in order to stabilize the resultant lipid systems. A typical process of forming liposomes is as follows:
1) dispersion of lipid or lipids and the phytosterols or phytostanols or mixtures thereof and the tocotrienol component in an organic solvent {such as chloroform, dichloromethane, ether, ethanol or other alcohol, or a combination thereof). A charged species may be added to reduce subsequent aggregation during liposome formation. Antioxidants (such as ascorbyl palmitate, alpha-tocopherol, butylated hydroxytoluene and butylated hydroxyanisole) may also be added to protect any unsaturated lipids, if present;
2) filtration of the mixture to remove minor insoluble components;
3) removal of solvents under conditions (pressure, temperature) to ensure no phase separation of the components occur,
4) hydration of the "dry" lipid mixture by exposure to an aqueous medium containing dissolved solutes, including buffer salts, chelating agents, cryoprotectorants and the like; and
5) reduction of liposome particle size and modification of the state of lamellarity by means of suitable techniques such as homogenization, extrusion etc..

Any procedure for generating and loading hydrated lipid with active ingredients, known to those skilled in the art, may be employed within the scope of this invention. For example, suitable processes for the preparation of liposomes are described in references 10 and 11, both of which are incorporated herein by reference. Variations on these processes are described in US Patent Serial No. 5,096,629 which is also incorporated herein by reference.

US Patent Serial No. 4,508,703 (also incorporated herein by reference) describes a method of preparing liposomes by dissolving the amphiphillic lipidic constituent and the hydrophobic constituent to form a solution and thereafter atomizing the solution in a flow of gas to produce a pulverent mixture.

### Cyclodextrin Complexes

Cyclodextrins are a class of cyclic oligosaccharide molecules comprising glucopyranose sub-units and having a toroidal cylindrical spatial configuration. Commonly available members of this group comprise molecules containing six (alpha-cyclodextrin), seven (beta-cyclodextrin) and eight (gamma-cyicodextrin) glucopyranose molcules, with the polar (hydrophilic) hydroxyl groups oriented to the outside of the structure and the apolar (lipophilic) skeletal carbons and ethereal oxygens fining the interior cavity of the toroid. This cavity is capable of accomodating (hosting) the lipophilic moiety of an active ingredient (the guest molecule, here the derivative of the present invention ) by bonding in a non-covalent manner to form an inclusion complex.

The external hydroxyl substituents of the cyclodextrin molecule may be modified to form derivatives having improved solubility in aqueous media along with other desired enhancements, such as lowered toxicity, etc.. Examples of such derivatives are: alkylated derivatives such as 2,6-dimethyl-beta-cyclodextrin; hydroxyalkylated derivatives such as hydroxypropyl-beta-cyclodextrin; branched derivatives such as diglucosly-beta-cyclodextrin; sulfoalkyl derivatives such as sulfobutylether-beta-cyclodextrin; and carboxymethylated derivatives such as carboxymethyl-beta-cylcodextrin. Other types of chemical modifications, known to those in the art, are also included within the scope of this invention.

The cyclodextrin complex often confers properties of improved solubility, dispersability, stability (chemical, physical and microbiological), bioavailability and decreased toxicity on the guest molecule (here, the derivative of the present invention).

There are a number of ways known in the art to produce a cyclodextrin complex. Complexes may be produced, for example, by using the following basic methods: stirring the one or more derivatves into an aqueous or mixed aqueous-organic solution of the cyclodextrin, with or without heating; kneading, slurrying or mixing the cyclodextrin and the present composition in a suitable device with the addition of an appropriate quantity of aqueous, organic or mixed aqueous-organic liquid, with or without heating; or by physical admixture the cylcodextrin and the composition of the present invention using a suitable mixing device. Isolation of the inclusion complex so formed may be achieved by co-precipitation, filtration and drying; extrusion/ spheronisation and drying; subdivision of the moist mass and drying; spray drying; lyophilization or by other suitable techniques depending on the process used to form the cyclodextrin complex. A further optional step of mechanically grinding the isolated solid complex may be employed.

These cyclodextrin complexes further enhance the solubility and dissolution rate and increase the stability of the derivatives. For a review of cyclodextrin complexation, please refer to 12.

### Complexation with Bile Salts

Bile acids, their salts and conjugated derivatives, suitably formulated, may be used to solubilize the derivatives of the present invention, thereby improving the solubility and dispersion characteristics of these compositions. Examples of suitable bile acids include: cholic acid, chenodeoxycholic acid, deoxycholic acid, dehydrocholic acid, and lithocholic acid. Examples of suitable bile salts include: sodium cholate, sodium deoxycholate and their other salt forms. Examples of suitable conjugated bile acids include: glycochenodeoxycholic acid, glycholic acid, taurochenodeoxycholic acid, taurocholic acid, taurodeoxycholic acid and their salts.

A suitable system for further enhancing the solubility of the derivative of the present invention consists of one or more derivatives plus one or more bile acids, salts or conjugated bile acids. Further materials may be added to produce formulations having additional solubilization capacity. These materials include, but are not limited to: phospholipids, glycolipids and monoglycerides. These ingredients may be formulated either in the solid phase or by the use of suitable solvents or carrier vehicles, with appropriate isolation and, optionally, particle size reduction using techniques described hereinabove.

Since bile acids and their derivatives have an unpleasant taste and may be irritating to the mucous membranes of the stomach and upper regions of the gastro-intestinal tract, a suitable enteric coating may be applied to the solid formulation particulates, using techniques known to those skilled in the art. Typical enteric coatings include, inter alia: cellulose acetate phthalate, cellulose acetate trimellitate, hydroxypropylmethylcellulose phthalate, hydroxypropylmethylcellulose acetate succinate, polyvinylacetate phthalate, acrylate polymers and their derivatives (e.g. appropriate members of the Eudragit™ series), ethylcellulose or combinations thereof. Additional excipients may be added to the coating formulation to modify membrane functionality or to aid in the coating process (e.g. surfactants, plasticisers, channeling agents, permeability modifiers and the like). Coating formulation vehicles may comprise aqueous or organic systems, or mixtures of both.

### Hydrotropic Complexation

Compounds which are capable of opening up the water structure associated with hydrophobic (lipophilic) and other molecules are referred to as hydrotropes. These compounds may be used to enhance further the aqueous solubility of the derivatives. Examples of hydrotopes include, inter alia, sodium benzoate, sodium hydroxybenzoates, sodium salicylate, nicotinamide, sodium nicotinate, sodium gentisate, gentisic acid ethanolamide, sodium toluates, sodium aminobenzoates, sodium anthranilate, sodium butylmonoglycolsulfate, resorcinol and the like.

Complex formation, which is non-covalent in nature, may be achieved by mixing one or more derivative and the hydrotope or mixtures thereof in a suitable liquid vehicle, which may be aqueous, organic or a combination of both. Additional excipients such as surfactants, polyols, disaccharides etc.. may be added to facilitate complexation or to aid in dispersability. The resultant complex is isolated as a dry powder by any process known in the art (co-precipitation and drying, evaporation of the liquid vehicle, spray drying, lyophilization etc..). Particle size may be reduced by any standard technique such as those described previously herein, if desired. The resultant hydrotope complex may be used without further modification or may be compounded into a variety of other formulations or vehicles as required.

### Methods of Use

The derivatives of the present invention may be administered to animals, in particular humans, directly and without further modification or may be treated to enhance further the solubility and/or dispersability of the composition as described in detail above. Alternatively, and optionally in conjunction with any one of these solubility and/or dispersability enhancement methods, the derivatives may be incorporated into various vehicles as described further below in order to treat and/or prevent CVD, its underlying conditions such as hypercholesterolemia, hyperlipidemia, arteriosclerosis, hypertension, thrombosis, related diseases such as Type II diabetes, as well as other diseases that include oxidative damage as part of the underlying disease process such as dementia, aging, and cancer. In populations, which are considered "high-risk" for CVD or any of the oxidation related disorders, it is contemplated that the derivatives of the present invention be used in primary, secondary and tertiary treatment programs.

Without limiting the generality of the foregoing, the derivatives of the present invention may be admixed with various carriers or adjuvants to assist in direct administration or to assist in the incorporation of the composition into foods, beverages, nutraceuticals or pharmaceuticals. In order to appreciate the various possible vehicles of the delivery of the derivatives, the list below is provided. The doses of the derivatives will vary depending upon, among other factors, the mode of delivery, the patient size and condition, the result to be achieved, as well as other factors known to those skilled in the art of food additives and medicinal agents. Generally, however, it is preferred that the derivatives of the present invention be administered to humans in a form comprising up to 6 grams of phytosterols and/or phytostanols per day. It will also be recognized that the provision of much larger daily doses of the derivatives are not harmful to the animal host, as excess will simply pass through normal excretory channels.

### 1) Pharmaceutical Dosage Forms:

It is contemplated within the scope of the present invention that the derivatives of the present invention may be incorporated into various conventional pharmaceutical preparations and dosage forms such as tablets (plain and coated) for use orally, bucally or lingually, capsules (hard and soft, gelatin, with or without additional coatings) powders, granules (including effervescent granules), pellets, microparticulates, solutions (such as micellar, syrups, elixirs and drops), lozenges, pastilles, ampoules, emulsions, microemulsions, ointments, creams, suppositories, gels, transdermal patches and modified release dosage forms together with customary excipients and/or diluents and stabilizers.

The derivatives of the present invention, adapted into the appropriate dosage form as described above may be administered to animals, including humans, orally, by injection (intravenously, subcutaneously, intra-peritoneally, intra-dermally or intra-muscularly), topically or in other ways. Although the precise mechanism of action is unclear, the derivatives of the present invention, administered intra-venously, lower serum cholesterol. It is believed that certain phytosterol-based products may have, in addition to the role as an inhibitors of cholesterol absorption in the intestine, a systemic effect on cholesterol homeostasis through bile acid synthesis, enterocycte and -biliary cholesterol excretion, bile acid excretion and changes in enzyme kinetics and cholesterol transport between various compartments within the body (PCT/CA97/00474 which was published on January 15, 1998). See also paper to Peter Jones (under publication).

### 2) Foods/Beverages/Nutraceuticals:

In another form of the present invention, the derivatives of the present invention may be incorporated into foods, beverages and nutraceuticals, including, without limitation, the following:
1 ) Dairy Products -such as cheeses, butter, milk and other dairy beverages, spreads and dairy mixes, ice cream and yoghurt;
2) Fat-Based Products-such as margarines, spreads, mayonnaise, shortenings, cooking and frying oils and dressings;
3) Cereal-Based Products-comprising grains (for example, bread and pastas) whether these goods are cooked, baked or otherwise processed;
4) Confectioneries-such as chocolate, candies, chewing gum, desserts, non-dairy toppings (for example Cool Whip™), sorbets, icings and other fillings;
5) Beverages- whether alcoholic or non-alcoholic and including colas and other soft drinks, juice drinks, dietary supplement and meal replacement drinks such as those sold under the trade-marks Boost™ and Ensure™; and
6) Miscellaneous Products-including eggs and egg products, processed foods such as soups, pre-prepared pasta sauces, pre-formed meals and the like.

The derivatives of the present invention may be incorporated directly and without further modification into the food, nutraceutical or beverage by techniques such as mixing, infusion, injection, blending, dispersing, emulsifying, immersion, spraying and kneading. Alternatively, the derivatives may be applied directly onto a food or into a beverage by the consumer prior to ingestion. These are simple and economical modes of delivery.

### EXAMPLES

The present invention is described by the following non-limiting examples:

### Example 1--Protection of ascorbic Acid

Oleum (24%, 8.3g) was added dropwise to acetone (50ml). Ascorbic acid (12g) was introduced to the mixture at 0°C, and the reaction mixture was stirred at 0°C for 6hrs. The obtained crystals were filtered off under suction, the filtered cake was pressed to dryness and then washed with acetone (30ml). The product, 5,6-isopropylidene-ascorbic acid (14g) was obtained.

### Example 2-- Attachment to Phytostanols

A solution of phytostanol mixture (24g) (campestanol: 36.4%; sitostanol: 62.3%) in toluene (500ml) and pyridine (25ml) was added dropwise to a mixture of phosphorus oxychloride (9ml) in toluene (200ml) at 0°C. The mixture was stirred at room temperature for 3hrs. The pyridine hydrochloride was filtered off and the mother liquor was concentrated to recover the toluene. The residue was dissolved in dry THF (100ml) and a solution of the above prepared protected ascorbic acid (14g) in dry THF (400ml) was added dropwise at 0°C. The stirring at room temperature was maintained for 1hr. The solution was concentrated to remove the solvent. Ethanol (400 ml) and 3N HCl (200ml) were added, the mixture was heated to 50°C for 30 min. and concentrated. Ethyl acetate (600ml) was added, the resulted solution was washed with water (3X300ml), dried over sodium sulfate, concentrated and the product (phytostanol-phosphate-ascorbate) was obtained as a white powder 22g.

### Example 3--Conversion to Sodium Salt

The above prepared acid (17g) was dissolved in ethanol (100ml) and a solution of sodium methoxide (2.7g) in ethanol (50ml) was added at stirring and at room temperature. The stirring was maintained for 30 min. After the addition. The resulted white cake was filtered off, dried and weighed, to afford a white powder 20g (phytostanol-phosphate-ascorbate sodium).

### Example 4―Effects of Derivative on Lipid Levels and Atherosclerotic Lesion Size in Apo-E Deficient Mice

### Background

Vitamin C (from Aldrich-Sigma) has been chemically attached to a blend of phytosterols/phytostanols comprising beta-sitosterol, sitostanol, campesterol and campestanol (referred to as "FCP-3P4"). The new derivative, which is the subject of the present invention, (VitoSterol or FCP-VP4) is water soluble. The effects of VitoSterol and its parent agents (3P4 and vitamin C) have been investigated in cholesterol-fed (0.2% w/w) male apo E-KO mice over 12 weeks. VitoSterol was administered by both water (2% w/v) and diets (2% w/w).

### Objective

- To test lipid-lowering effects of VitoSterol in apo E-KO mice
- To test anti-atherogenic effects of Vitosterol in apo E-KO mice

### Specific aims

1. To test the effects of VitoSterol by either dietary or drinking water route of administration on plasma cholesterol and triglycerides levels in apo E-KO mice.
2. To evaluate the compliance of VitoSterol especially in drinking water and its effects on body weight of mice.

### Materials and Methods

Forty eight 4-week male apolipoprotein E knockout (apo E-KO) mice were purchased from the Jackson Laboratory and housed in the animal facility at Research Center, BC Children's Hospital. VitoSterol was prepared by Forbes Medi-Tech Inc. ("FMI") The parent material of VitoSterol, phytosterols (Forbes Batch # 98-694) and vitamin C (purchased from Aldrich-Sigma) was provided by FMI. Cholesterol was purchased from Sigma and 9% (w/w) PicoLab mouse chow was purchased from Jameison's Pet Food distributor, Delta, BC. Laboratory chemicals and reagents were prepared and used as previously described (13-16).

### Experimental design:

**Animals and diets:** Based on our own previous experiments, 8 animals in each group are sufficient to detect statistically significant treatment-related effects on plasma cholesterol levels and development of atherosclerotic lesions. Thus, as outlined below, 48 male apo E-KO mice were divided into 6 groups of 8 with similar mean plasma cholesterol level and body weight. The animals were fed with the experimental diets for 14 weeks. Diet preparation was carried out based on previously published methods (13-16). The outline of experimental groups of mice and diets is presented in Table 1. The animals were bled from the tail at base line and during the experiment for measurement of plasma lipid levels. At the end of the study, the mice were sacrificed using CO₂ gas and taking blood from the heart. The hearts and aorta were removed and fixed in formalin for the assessment of atherosclerotic lesions. Aliquots of plasma were sent to BRI laboratory, Burnaby, BC, for measurement of vitamin C and plant sterol concentrations. Aliquots of plasma were sent to Dr. Kitts laboratory, UBC, for antioxidant evaluations.

**Table 1:**

| Experimental groups of mice and diets | |
|---|---|
| **Groups (n)** | **Treatment (types of diets)** |
| 1 (8) | Control mouse chow with 9% fat & 0.15% (w/w) cholesterol (MC) |
| 2 (8) | MC+2% (w/w) VitoSterol |
| 3 (8) | MC+ 2% (w/v) VitoSterol in drinking water |
| 4 (8) | MC+ 1.3% (w/w) phytosterols (equivalent amount to that which is given to group 2) |
| 5 (8) | MC+ 0.7% (w/w) vitamin C (equivalent amount to that which is given to group 2) |
| 6 (8) | MC+ 1.3% (w/w) phytosterols and 0.7% (w/w) vitamin C (equivalent amounts to those which are given to group 2) |

**Biochemical and histological assessments:** Plasma cholesterol levels were estimated at baseline and at 4-6 week intervals during the experiment, and at the end of the experiment as previously described (13-16) in the clinical laboratory at St. Paul's Hospital. Aliquots of plasma samples were used for determination of vitamin C and plant sterol concentrations using liquid chromatography techniques in BRI. Aortic root sections were used for evaluation of atherosclerotic lesion development as previously described (13-14).

### Results

### A: Plasma total cholesterol levels

Figure 1 shows the effects of VitoSterol and its parent agents on plasma total cholesterol levels. Both water solution and dietary VitoSterol had a marked cholesterol lowering effect. This marked effect was consistent over the 12 weeks of the study.

### B: Plasma triglycerides

Figure 2 shows the effects of the compounds on plasma triglyceride levels. VitoSterol had a slight TG-lowering effects. As is evident, at week 12 the triglyceride effects of VitoSterol was diminished.

### C: Body Weight

Figure 3 shows body weight. Decreased body weight in dietary VitoSterol group was probably due to diet preparation. The drop in body weight seems to be related to food intake and diet preparation.

### D: Fecal cholesterol levels

Preliminarily results show a marked inhibition of cholesterol absorption by VitoSterol. The results summarized below:

| Group | Fecal cholesterol (%W/W) |
|---|---|
| Control | 0.12 |
| Dietary VitoSterol | 0.8 |
| Water VitoSterol | 0.7 |
| 3P4 | 0.2 |

### E: Atherosclerotic Lesion Size

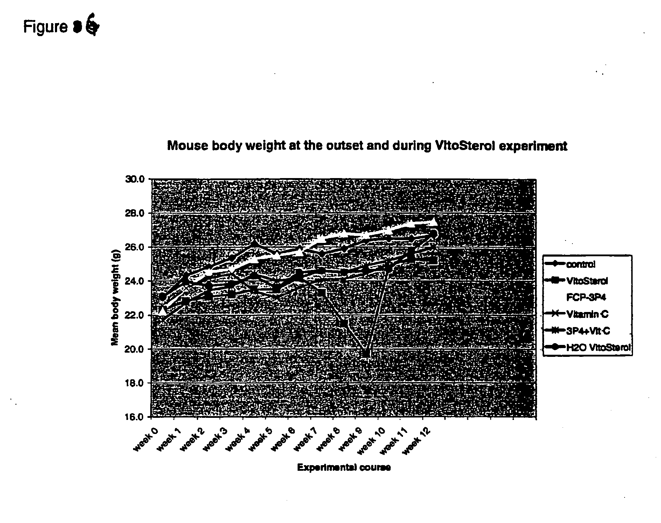

Figure 4 shows that FM-VP4 reduces atherosclerotic lesion size by 75% as compared to the control group. The treatment groups were numbered in the following manner:

**Table 2:**

| Experimental groups of mice and diets for lesion size study | |
|---|---|
| **Groups (n)** | **Treatment (types of diets)** |
| 1 (8) | Control mouse chow with 9% fat & 0.2% (w/w) cholesterol (MC) |
| 2 (8) | MC+ 0.2% cholesterol + 2% (w/w) VitoSterol (i.e. in food) |
| 3 (8) | MC+ 0.2% (w/w) cholesterol and 1.2% (w/w) phytosterols |
| 4 (8) | MC+ 0.2%(w/w) cholesterol and 0.5% (w/w) vitamin C |
| 5 (8) | MC+ 0.2% (w/w) cholesterol + 1.2% phytosterols+0.5% vitamin C |
| 6 (8) | MC+ 0.2% (w/w) cholesterol + 2% Vitosterol (w/v) in water |

### Conclusions

VitoSterol is a potent cholesterol-lowering agent with an additional triglyceride-lowering effect. We observed no apparent side effects over the 12 weeks of experiment. Vitosterol was found to reduce cholesterol levels by 75% and triglyceride levels by 44%. These are excellent results. In addition, dramatic results on atherosclerotic lesion size were observed (71 to 86% reduction in Vitosterol (new derivative) treatment groups as compared to the control group).

### REFERENCES

1. Law M.R., Wald N.J., Wu., Hacksaw ZA., Bailey A.; Systemic underestimation of association between serum cholesterol concentration and ischemic heart disease in observational studies: Data from BUPA Study; *Br. Med. J.* 1994; 308:363-366
2. Law M.R., Wald N.J., Thompson S.G.; By how much and how quickly does reduction in serum cholesterol concentration lower risk of ischemic heart disease? *Br. Med. J.* 1994; 308:367-373
3. La Rosa J.C., Hunninghake D.. Bush D. et al.; The cholesterol facts: A summary of the evidence relating to dietary fats, serum cholesterol and coronary heart disease:Ajoint statement by the American Heart Association and the National Heart, Lung and Blood Institute. Circulation 1990; 81:1721-1733
4. Havel R.J., Rapaport E.. Drug Therapy: Management of Primary Hypedipidemia. New *England Journal of Medicine*, 1995; 332:1491-1498
5. Kuccodkar et al.; Effects of plant sterols on cholesterol metabolism. *Atherosclerosis,* 1976; 23:239-248
6. Lees R.S., Lees A.M. Effects of sitosterol therapy on plasma lipid and lipoprotein concentrations. In: Greten H (Ed) Lipoprotein Metabolism. Springer-Verlag, Berlin, Heidelberg, New York, 1976:119-124
7. Lees A.M., Mok H.Y.I., Lees R.S., McCluskey M.A., Grundy S.M. Plant sterols as cholesterol-lowering agents: clinical trials in patients with hypercholesterolemia and studies of sterol balance. Atherosclerosis 1977; 28: 325-338
8. Attwood D. Micoremulsions. In Colloidal Drug Delivery Systems (J. Kreuter ed.) Marcel Dekker, New York, 1994:32
1. Eugster C., Rivara G., Fomi G and Vai S. Marigenol Concentrates comprising Taxol andor Taxan esters as active substances. *Panmimerva* Med, 199638:234-242
10. Liposome Drug Delivery Systems, Technomic Publishing Co. Inc., Lancaster, PA 1993
11. Pharmaceutical Technology: Liposomes as Drug Delivery Systems Parts I, II and III, October 1992, November 1992 and January 1993 respectively.
12. Rajewski R.A. and Valentino J.S. Pharmaceutical Applications of Cyclodextrins/In vivo Drug Delivery System. *J. Pharm. Sci*. 1996: 85:1142-1169
13. Moghadasian et al. Pro-atherogenic and anti-atherogenic effects of probucol and phytosterols in apo E knockout mice: Possible mechanism of action. Circulation 1999;99:1733-1739.
14. Moghadasian et al. "Tall oil"-derived phytosterols reduce atherosclerosis in apo E-deficient mice. Arterioscler Thromb Vase Biol 1997;17:119-126.
15. Moghadasian et al. Histologic, hematologic, and biochemical characteristics of apo E-deficient mice: effects of dietary cholesterol and phytosterols. Lab invest 1999;79:355-364.
16. Moghadasian et al. Lack of regression of atherosclerotic lesions in phytosterol-treated apo E-deficient mice. Life Sci 1999;64:1029-10

## Claims

1. Use of a compound comprising phytosterol and phytostanol and ascorbic acid, including salts thereof, and represented by the general formulae: 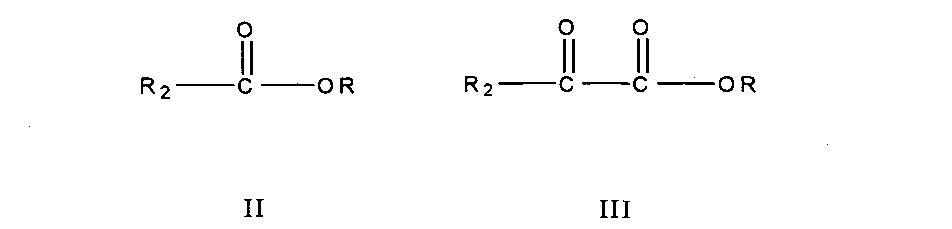 wherein R is a phytosterol or phytostanol moiety; and is an ascorbic acid or a protected ascorbic acid group, in the manufacture of a composition for use in a method of treatment or prevention of cardiovascular disease in an animal.

2. Use according to claim 1 in which the disease is atherosclerosis or hypercholesterolemia.

3. Use of claim 1 or claim 2 wherein the animal is human.

4. Use according to any preceding claim wherein the phytosterol is selected from the group consisting of sitosterol, campesterol, stigmasterol, brassicasterol, desmosterol, chalinosterol, poriferasterol, and clionasterol.

5. Use according to any of claims 1 to 3 wherein the phytostanol is selected from the group consisting of all saturated or hydrogenated phytosterols.

6. Use according to any of claims 1 to 3 wherein the phytostanol is sitostanol.

7. Use according to any of claims 1 to 3 wherein the compound has the formula II.

8. Use according to any of claims 1 to 3 wherein the compound has the formula III.

9. A pharmaceutical, comestible or verage composition which comprises a compound comprising phytosterol and phytostanol and ascorbic acid, including salts thereof, and represented by the general formulae: 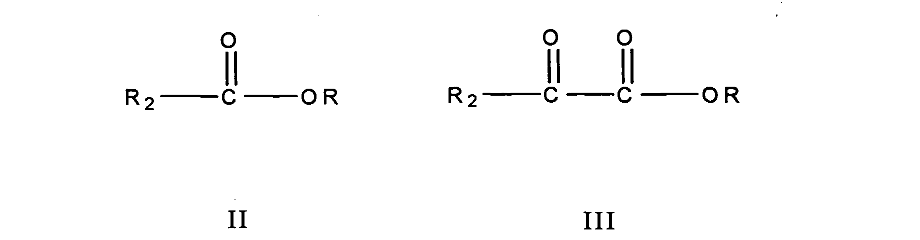 wherein R is a phytosterol or phytostanol moiety; and is an ascorbic acid or a protected ascorbic acid group.

10. A composition according to claim 9 wherein the phytostanol is selected from the group consisting of all saturated or hydrogenated phytosterols.

11. A composition according to claim 9 wherein the phytostanol is sitostanol.

12. A composition according to claim 9 wherein the compound has the formula II.

13. A composition according to claim 9 wherein the compound has the formula III.

14. A composition according to claim 9 which is a pharmaceutical composition additionally comprising at least one agent which reduces cholesterol synthesis in humans.

15. A composition according to claim 14 wherein the agent is a 3-hydroxy-3-methyl glutaryl coenxyme-A (HMG-CoA) reductase inhibitor.

16. A compound comprising phytosterol or phytosterol and ascorbic acid having one of the following formulae: 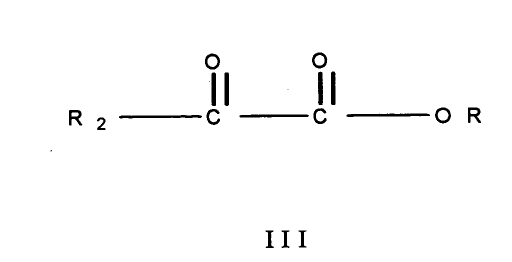 wherein R is a phytosterol or phytostanol moiety; and is an ascorbic acid or a protected ascorbic acid group.

17. A compound according to claim 16 wherein the phytosterol is selected from the group consisting of sitosterol, campesterol, stigmasterol, brassicasterol, desmosterol, chalinosterol, poriferasterol, and clionasterol.

18. A compound according to claim 16 wherein the phytostanol is selected from the group consisting of all saturated or hydrogenated phytosterols.

19. A compound according to claim 16 wherein the phytostanol is sitostanol.
